# EUROPÄISCHE PATENTSCHRIFT

(11) **EP 2 542 780 B1**
(45) Veröffentlichungstag und Bekanntmachung des Hinweises auf die Patenterteilung: **18.12.2013**
(21) Anmeldenummer: 10801417.6
(22) Anmeldetag: 23.12.2010
(51) Int. Cl.: F04B 43/12, F04B 43/08, A61M 5/142

(54) **SCHLAUCHPUMPE**
PERISTALTIC PUMP
POMPE PÉRISTALTIQUE

(30) Priorität: 01.03.2010 DE 102010000591
(43) Veröffentlichungstag der Anmeldung: 09.01.2013
(73) Patentinhaber: Ulrich GmbH & Co. KG, 89081 Ulm (DE)
(72) Erfinder: ZUPP, Andre, 06847 Dessau (DE); SCHWERDTFEGER, Uwe, 39439 Amesdorf (DE)
(74) Vertreter: Charrier, Rapp & Liebau
(86) Internationale Anmeldenummer: PCT/EP2010/070713
(87) Internationale Veröffentlichungsnummer: WO 2011/107178

(56) Entgegenhaltungen:
- FR-A5- 2 157 184
- US-A- 4 178 138
- US-A1- 2002 071 776

## Beschreibung

Die Erfindung betrifft eine Schlauchpumpe nach dem Oberbegriff des Anspruchs 1.

Derartige Schlauchpumpen werden insbesondere im Bereich der Medizintechnik, beispielsweise als Infusionspumpe oder in Injektions- und Dialysegeräten verwendet. Aus der US 2002/071 776 A1, die als nächstliegender Stand der Technik betrachtet ist, ist eine peristaltische Pumpe zur Förderung eines in einem Schlauch geführten Mediums bekannt, wobei der Schlauch mittels Quetschrollen gegen die Wirkfläche eines Gegenlagers gedrückt wird, um das Medium in dem Schlauch in Förderrichtung weiterzufördern. Der Abstand zwischen den Quetschrollen und der Wirkfläche des Gegenlagers ist dabei in zwei verschiedenen Einstellungen einstellbar, um die Pumpe in eine Ausgangsstellung zu bringen, in der ein Schlauch zwischen den Quetschrollen und dem Gegenlager eingebracht werden kann und anschließend in eine Pumpstellung zu verstellen, in der die Quetschrollen den Schlauch gegen die Wirkfläche des Gegenlagers drücken.

Aus der FR 2 157 184 A5 ist eine weitere Schlauchpumpe bekannt, bei der ein Schlauch mittels eines Quetschelements gegen ein Gegenlager gedrückt wird, wobei die Wirkfläche des Gegenlagers durch den Innenumfang eines Kreisrings gebildet ist.

Eine weitere Schlauchpumpe ist beispielsweise aus der Patentschrift AT 367874 bekannt. Darin ist eine Schlauchpumpe mit mehreren über ein Planetengetriebe von einem Zentralteil antreibbaren Rollen beschrieben, bei der die Rollen auf zumindest einem Schlauch, in dem das zu fördernde Medium geführt wird, unter Quetschung von dessen freiem Querschnitt abrollen. Die Rollen sind drehbar auf einem rotierenden Träger mit größerem Spiel gelagert und stehen mit zumindest einem Teil ihres Umfangs während des Anliegens an dem Schlauch mit dem Zentralteil in reibungsschlüssigem Kontakt. Die rotierende Bewegung des Trägers und der Rollen führt dazu, dass sich die Quetschstelle entlang des Schlauches bewegt wodurch das in dem Schlauch geführte Medium in Förderrichtung vorangetrieben wird.

Durch die alternierende Quetschung wird der Schlauch bei laufender Pumpe einer erheblichen mechanischen Belastung ausgesetzt. Insbesondere bei auftretendem Schlupf zwischen der Oberfläche der Rollen und der Schlauchoberfläche treten massive Pressungen und Scherkräfte auf, welche den Schlauch strecken und ziehen. Bei hohem Druck kann sich der Schlauch daher aufblähen oder sogar zerstört werden. Um den Schlauch weitgehend zu schonen und den Justieraufwand beim Einführen des Schlauchs in die Schlauchpumpe möglichst gering zu halten, ist bei der aus der AT 367874 bekannten Schlauchpumpe ein den Schlauch gegen mindestens eine der Rollen drückender Gegendruckkörper vorgesehen, welcher in Bezug auf den Zentralteil der Schlauchpumpe in radialer Richtung verschiebbar auf einer Grundplatte gehalten und mittels einer Feder in Richtung zum Zentralteil vorgespannt ist. Dadurch wird erreicht, dass beim Austausch eines Schlauchs lediglich ein neuer Schlauch einzulegen ist und dass bereits durch die Vorspannung des Gegendruckkörpers mittels der Feder eine ausreichende Quetschung durch die Rollen sichergestellt ist, um die Funktionsfähigkeit der Pumpe zu gewährleisten. Weiterhin werden Toleranzen in der Pumpenanordnung und den Schläuchen durch den federnden Gegendruckkörper ausgeglichen.

Diese aus dem Stand der Technik bekannte Anordnung hat sich jedoch als störanfällig erwiesen, weil die Federn, welche die Vorspannung des Gegendruckkörpers erzeugen, im Laufe der Zeit ihre Spannkraft verlieren oder bei Materialermüdung sogar brechen können. Der Austausch der Feder ist allerdings umständlich und zeitaufwendig. Ferner variiert der Abstand zwischen den Rollen und dem Gegenlager über dessen Umfang, woraus ein geringerer Pumpendruck resultiert.

Hiervon ausgehend liegt der Erfindung die Aufgabe zugrunde, eine Schlauchpumpe aufzuzeigen, bei der Fertigungstoleranzen der Pumpe und Materialungleichheiten des Schlauchs möglichst gut ausgeglichen und die mechanische Belastung des Schlauchs beim Betrieb der Pumpe möglichst gering gehalten werden können, wobei gleichzeitig ein möglichst hoher Pumpendruck bei geringer Belastung des Schlauchs und eine möglichst wartungsarme Handhabung der Schlauchpumpe gewährleistet werden soll. Insbesondere soll der Aufwand zur Einstellung und Justierung der Schlauchpumpe während bzw. unmittelbar nach ihrer Fertigung und bei regelmäßigen Wartungen reduziert werden.

Diese Aufgaben werden mit einer Schlauchpumpe mit den Merkmalen des Anspruchs 1 gelöst. Bevorzugte Ausführungsformen dieser Schlauchpumpe sind den Unteransprüchen zu entnehmen.

Die erfindungsgemäße Schlauchpumpe umfasst ein Gehäuse und mehrere Quetschelemente, welche bevorzugt als Quetschrollen ausgebildet sind und den Schlauch unter Quetschung gegen die Wirkfläche eines Gegenlagers drücken, um dadurch das in dem Schlauch geführte Medium in Förderrichtung weiter zu fördern. Der Abstand zwischen den Quetschelementen und der Wirkfläche des Gegenlagers ist dabei variabel. Um eine geeignete und den Schlauch möglichst wenig belastende Einstellung des Abstands zwischen den Quetschelementen und der Wirkfläche zu ermöglichen, ist nach der Erfindung vorgesehen, dass das Gegenlager über eine konische Stellfläche verfügt, welche sich gegen eine komplementär geformte Stützfläche am Gehäuse abstützt. Der Abstand zwischen den Quetschelementen und der Wirkfläche des Gegenlagers kann dabei durch Verschieben des Gegenlagers gegenüber dem Gehäuse entlang der Stützfläche eingestellt werden. Durch die Verstellbarkeit des Abstands zwischen den Quetschelementen und der Wirkfläche des Gegenlagers können etwaige Fertigungstoleranzen, die bei der Herstellung der Pumpe unweigerlich auftreten, sowie etwaige Materialtoleranzen im Schlauchkörper ausgeglichen werden. Der Abstand zwischen den Quetschelementen und der Wirkfläche wird dabei erstmalig noch vor der ersten Ingebrauchnahme der Schlauchpumpe und gegebenenfalls später bei Wartungsarbeiten so eingestellt, dass eine Mindestpressung des Schlauches gewährleistet wird, bei der die Schlauchpumpe funktionsfähig ist und die erforderlichen Betriebsparameter und Arbeitspunkte erreicht werden können. Eine Überpressung des Schlauches findet allenfalls im Rahmen zulässiger Schlauchtoleranzen statt. Gleichzeitig wird die mechanische Belastung auf das gesamte Pumpensystem reduziert und die Leistungsaufnahme in den Arbeitspunkten ist weitgehend konstant, so dass eine relativ gleichbleibende elektrische Leistungsaufnahme erzielt werden kann. Insgesamt kann dadurch die Lebensdauer sowohl der Schlauchpumpe selbst als auch der als Verbrauchsmaterial eingesetzten Schläuche verlängert werden.

In einer bevorzugten Ausführungsform der erfindungsgemäßen Schlauchpumpe ist vorgesehen, dass das Gegenlager an einen Stellring gekoppelt ist, welcher auf einem Außengewinde am Gehäuse der Schlauchpumpe geführt ist und über Verdrehen gegenüber dem Gehäuse in axialer Richtung relativ zum Gehäuse zwischen einer oberen Grenzstellung und einer unteren Grenzstellung verschiebbar ist. An dem Stellring liegt ein Verschiebering an, der bei axialer Verschiebung des Stellrings gegenüber dem Gehäuse ebenfalls in axialer Richtung verschoben wird. An dem Verschiebering ist ein Druckring befestigt, welcher wiederum mit dem Gegenlager verbunden ist. Bei Verdrehen des Stellrings gegenüber dem Gehäuse bewegt sich daher die konische Stellfläche des Gegenlagers auf der Stützfläche des Gehäuses und zieht das Gegenlager bezüglich des Gehäuses nach innen bzw. nach außen, je nach Drehrichtung des Stellrings. Wird der Stellring in Richtung seiner unteren Grenzstellung verschoben, so wird das Gegenlager in das Innere des Gehäuses gezogen und bei umgekehrter Drehrichtung des Stellrings nach außen verschoben. Das Gegenlager und der daran befestigte Druckring sind bevorzugt als Ringsegment ausgebildet. Beim Verschieben der konischen Stellfläche des Gegenlagers entlang der Stützfläche des Gehäuses verändert sich der Durchmesser des als Ringsegment ausgebildeten Gegenlagers, wodurch sich auch das Spaltmaß, d.h. der Abstand zwischen der Wirkfläche des Gegenlagers und den Quetschelementen (insbesondere der Außenumfangsfläche der Quetschrollen) ändert. Durch ein Verdrehen des Stellrings gegenüber dem Gehäuse ist dadurch das Spaltmaß auf den optimalen Wert einstellbar. Zur Einstellung des Spaltmaßes wird zweckmäßig eine Lehre verwendet, die beim Einstellvorgang zwischen die Wirkfläche des Quetschelements (also insbesondere dem Außenumfang der Quetschrollen) und der Wirkfläche des Gegenlagers eingelegt wird.

Nachfolgend wird die Erfindung anhand eines Ausführungsbeispiels unter Bezugnahme auf die begleitenden Zeichnungen näher erläutert. Die Zeichnungen zeigen:
- **Figur 1:**: Draufsicht auf eine Injektionsvorrichtung, in der eine erfindungsgemäße Schlauchpumpe verwendet wird;
- **Figur 2:**: Perspektivische Darstellung einer erfindungsgemäßen Schlauchpumpe;
- **Figur 3:**: Explosionsdarstellung der Schlauchpumpe von Figur 2;
- **Figur 4:**: Schnittdarstellung der Schlauchpumpe von Figur 2 entlang der Ebene A-A;
- **Figur 5:**: Detailansicht der Schnittdarstellung von Figur 4 im Bereich des Gegenlagers und einer dem Gegenlager gegenüberliegenden Führungsrolle;
- **Figur 6:**: Detailansicht einer Querschnittsdarstellung der Schlauchpumpe von Figur 2 im Bereich des Gegenlagers und einer dem Gegenlager gegenüberliegenden Quetschrolle, wobei Figur 6a das Gegenlager in seiner ersten (oberen) Grenzstellung und Figur 6b das Gegenlager in seiner zweiten (unteren) Grenzstellung zeigt;

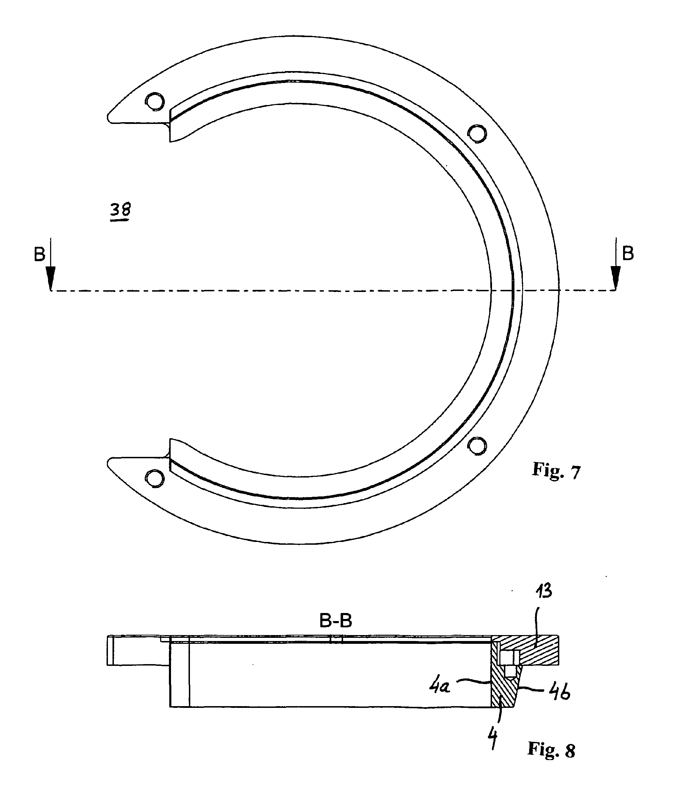

- **Figur 7:**: Draufsicht auf das Gegenlager der Schlauchpumpe von Figur 2 und dem mit dem Gegenlager verbundenen Druckring;
- **Figur 8:**: Schnittdarstellung des Gegenlagers und des damit verbundenen Druckrings von Figur 7 entlang der Ebene B-B.

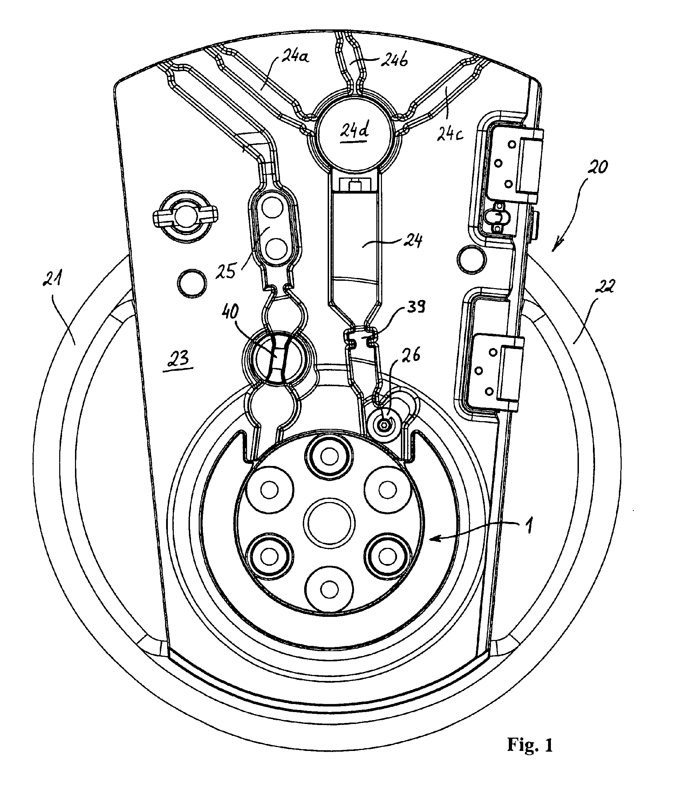

In Figur 1 ist der Injektionskopf einer Injektionsvorrichtung zur Injektion von zwei verschiedenen oder gleichen Kontrastmitteln und einer NaCl-Spüllösung in die Blutbahn eines Patienten gezeigt, in der eine erfindungsgemäße Schlauchpumpe 1 verwendet wird. Solche Injektionsvorrichtungen werden bspw. zur Injektion von Kontrastmitteln bei der Durchführung von bildgebenden Verfahren wie Computertomographie, Ultraschalluntersuchungen und Computertomographie (MRT) verwendet. Die Injektionsvorrichtung umfasst den in Figur 1 gezeigten Injektionskopf 20, in dem die Schlauchpumpe 1 angeordnet ist. Der Injektionskopf 20 umfasst ein Kunststoffgehäuse mit zwei kreisringförmigen Handgriffen 21, 22. Zwischen den Handgriffen 21 und 22 ist ein Panel 23 angeordnet, welches mit einem hier zeichnerisch nicht dargestellten Deckel verschließbar ist. Das Panel 23 weist in seinem unteren Bereich eine Ausnehmung zur Aufnahme der Schlauchpumpe 1 auf. Darüber befinden sich kanalförmige Ausnehmungen 24, 25, in welche eine verzweigte Schlauchanordnung (welche hier zeichnerisch nicht dargestellt ist) eingefügt werden kann. Bei der Schlauchanordnung handelt es sich insbesondere um eine Schlauchanordnung, wie sie in der EP 2 011 541 A2 im Detail beschrieben ist. Diese Schlauchanordnung umfasst insgesamt drei Zufuhrschläuche, nämlich einen ersten Zufuhrschlauch für ein erstes Kontrastmittel, einen zweiten Zufuhrschlauch für ein zweites Kontrastmittel und einen dritten Zufuhrschlauch für eine Spüllösung (insbesondere NaCl). Die drei Zufuhrschläuche werden an hier ebenfalls zeichnerisch nicht dargestellte Vorratsflaschen für die Kontrastmittel und die Spüllösung angeschlossen und in die im oberen Bereich des Panels 23 angeordneten Verzweigungen 24a, 24b und 24c der Ausnehmung 24 eingefügt. Die drei von den Vorratsbehältern kommenden Zufuhrschläuche werden über ein Verzweigungsstück, welches in der kreisförmigen Ausnehmung 24d des Panels 23 eingefügt wird, in einem Schlauchstück zusammengeführt, welches zur Schlauchpumpe 1 geführt wird.

Zur Einführung des Schlauchs in die Schlauchpumpe 1 ist eine Einfädeleinrichtung vorgesehen. Die Funktionsweise und die Ausgestaltung dieser Einfädeleinrichtung wird im Folgenden noch erläutert. Der Schlauch wird schließlich durch die Schlauchpumpe 1 geführt und in die Ausnehmung 25 im oberen, linken Teil des Panels 23 eingelegt. Das Schlauchende wird an einen Patientenschlauch angeschlossen, über den die in dem Schlauch geführten Medien schließlich in die Blutbahn des Patienten injiziert werden können. Zur Fixierung des Schlauchs auf dem Panel 23 ist eine Fixiereinrichtung vorgesehen, welche eine Fixierung des Schlauchs an einer ersten, eingangsseitigen Stelle 39 und wenigstens einer zweiten, ausgangseitigen Stelle 40 der Schlauchpumpe ermöglicht. An den Fixierstellen 39 und 40 sind zweckmäßig Ultraschallsensoren zur Erfassung von Luftblasen in dem Schlauch angeordnet. Weitere Fixierstellen des Schlauchs auf dem Panel 23 sind möglich und bspw. in der EP 2011541 A1 beschrieben.

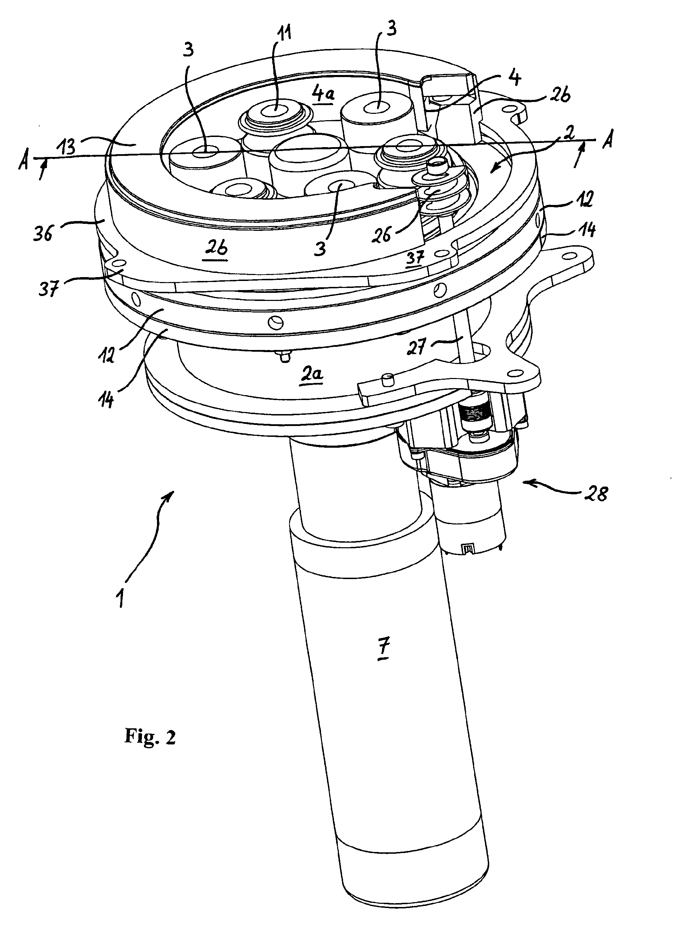

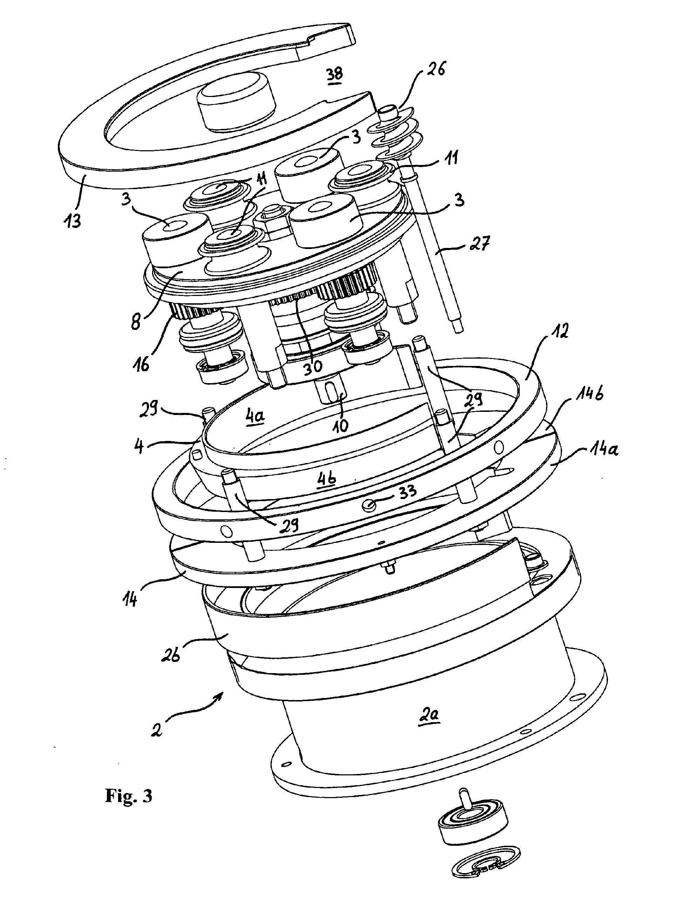

In den Figuren 2 und 3 ist die Schlauchpumpe 1 im Detail in einer perspektivischen Ansicht gezeigt, wobei Figur 3 eine Explosionsdarstellung ist. Die Schlauchpumpe 1 umfasst eine untere Pumpeneinheit mit einem Antriebsmotor 7, sowie eine obere Pumpeneinheit mit einem Gehäuse 2. Das Gehäuse 2 ist in einen unteren Gehäuseteil 2a und einen oberen Gehäuseteil 2b unterteilt. Der untere Gehäuseteil 2a kann mit dem oberen Gehäuseteil 2b einstückig oder auch zweiteilig ausgebildet sein.

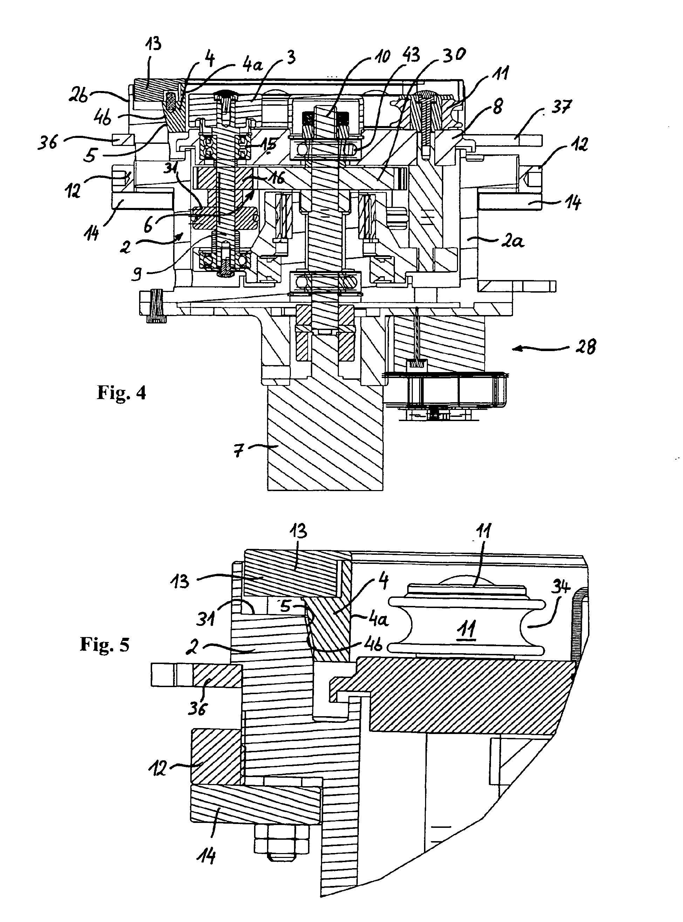

Die untere Pumpeneinheit umfasst den Antriebsmotor 7 mit einer Antriebswelle 10, welche über ein Getriebe mit der oberen Pumpeneinheit gekoppelt ist. Der Aufbau der oberen Pumpeneinheit ist aus der Schnittdarstellung der Figur 4 zu entnehmen. Im Innern des Gehäuses 2 ist ein an die Antriebswelle 10 des Antriebsmotors 7 gekoppeltes Getriebe 6 angeordnet. Das Getriebe umfasst ein Sonnenrad 30, welches drehfest mit der Antriebswelle 10 des Antriebsmotors 7 verbunden ist. Das obere Ende der Antriebswelle 10 ist über ein Lager 43 in einer Trägerscheibe 8 drehbar gelagert. Auf der Trägerscheibe 8 sind mehrere Quetschelemente 3 angeordnet. Bei den Quetschelementen 3 handelt es sich bei dem hier zeichnerisch dargestellten Ausführungsbeispiel um angetriebene Quetschrollen 3, wobei hier drei solcher Quetschrollen 3 gleichförmig am Außenumfang der kreisrunden Trägerscheibe 8 angeordnet sind. Die Quetschrollen 3 sind drehbar auf der Trägerscheibe 8 gelagert. Hierfür ist jede der drei Quetschrollen 3 auf einer Welle 9 mit einer Achse 9' aufgesetzt und jede Welle 9 ist über ein Lager 15 in einer Bohrung der Trägerscheibe 8 gelagert. Die Wellen 9 und damit die Achsen 9' der Quetschrollen 3 verlaufen parallel zur Antriebswelle 10 des Antriebsmotors 7. Der Antriebsmotor 7 versetzt die Trägerscheibe 8 und die Quetschrollen 3 bei laufender Pumpe über das Getriebe 6 in Drehung. Das Getriebe 6 umfasst neben dem Sonnenrad 30 Planetenräder 16, wobei jeder Quetschrolle 3 ein solches Planetenrad 16 zugeordnet und drehfest an der Welle 9 befestigt ist. Jedes der Planetenräder 16 ist über eine Zahnung an das Sonnenrad 30 des Planetengetriebes gekoppelt. An jeder Welle 9 ist neben dem Planetenrad 16 ein Reibrad 31 angeordnet, wobei das Reibrad 31 drehfest und im Abstand zum Planetenrad 16 an der Welle 9 befestigt ist. Am Außenumfang jedes Reibrads 31 ist eine umlaufende Nut angeordnet, in welche ein Gummiring 32 (O-Ring) eingefügt ist. Über diesen Gummiring 32 steht das Reibrad 31 mit dem Innenumfang 2c des Pumpengehäuses 2 in Kontakt. Der Innenumfang 2c des Gehäuses 2 wirkt dadurch als Hohlrad eines Planetengetriebes. Wird die Antriebswelle 10 durch den Antriebsmotor 7 in Rotation versetzt, so wird diese Rotationsbewegung über die Kopplung des Planetenrads 16 am Sonnenrad 30 auf die Welle 9 übertragen, wodurch sich die Welle 9 und die drehfest mit dieser verbundene Quetschrolle 3 in Rotation versetzt. Gleichzeitig rollt das Reibrad 31 am Innenumfang 2c des Pumpengehäuses 2 ab, wodurch sich die Trägerscheibe 8 ebenfalls gegenüber dem Pumpengehäuse 2 in Drehung versetzt. Durch die Reibräder 31 kann die Trägerscheibe 8 von dem Antriebsmotor 7 auch dann in Rotation versetzt werden, wenn sich noch kein Schlauch in der Schlauchpumpe befindet.

Auf der Trägerscheibe 8 sind zusätzlich zu den Quetschrollen 3 noch Führungsrollen 11 gelagert. Die Führungsrollen 11 dienen zur Führung des Schlauchs zwischen benachbarten Quetschrollen 3 und sind nicht angetrieben. Am Außenumfang weisen die Führungsrollen 11 eine im Querschnitt halbkreisfömlige Nut 34 auf, in welcher der Schlauch geführt wird (Figur 5). Die Anordnung der Führungsrollen 11 und der Quetschrollen 3 auf der Trägerscheibe 8 ist insbesondere der Explosionsdarstellung der Figur 3 zu entnehmen.

Zur Einführung des Schlauchs in die Schlauchpumpe ist eine Einfädeleinrichtung vorgesehen, welche den Schlauch selbsttätig zwischen den Quetschrollen 3 und dem Gegenlager 4 einfädelt. Die Einfädeleinrichtung umfasst eine außerhalb der Trägerscheibe 8 angeordnete Schneckenspindel 26. Die Schneckenspindel 26 ist auf einer Welle 27 angeordnet, wobei die Welle 27 parallel zur Achse 9' der Quetschrollen 3 verläuft. Die Welle 27 ist drehbar in einem Gehäuseteil 2 der Schlauchpumpe gelagert und an einen Spindelantrieb 28 gekoppelt, mit dem die Welle 27 und die Schneckenspindel 26 in Drehung versetzt werden kann, um einen in die Schneckenspindel eingelegten Schlauch in die Schlauchpumpe einzufädeln. Die oberen Schneckengänge der Schneckenspindel 26 ragen in Längsrichtung der Schlauchpumpe (also parallel zur Achse der Wellen 10 bzw. 27) über die Oberseite der Quetschrollen 3 und der Führungsrollen 11 hinaus.

Am oberen Ende der oberen Pumpeneinheit ist ein Gegenlager 4 angeordnet. Das Gegenlager 4 ist kreissegmentförmig mit einer Aussparung 38 ausgebildet und erstreckt sich zweckmäßig über einen Winkelbereich von 200° bis 300°. Die Schneckenspindel 26 ist im Bereich der Aussparung 38 des Gegenlagers 4 angeordnet. Das Gegenlager 4 verfügt über eine Wirkfläche 4a, welche dem Außenumfang der Quetschrollen 3 in einem Abstand d gegenüberliegt. In dem Spalt zwischen der Wirkfläche 4 und dem Außenumfang jeder Quetschrolle 3 wird der Schlauch eingefädelt.

Zum Einführen des Schlauchs in die Schlauchpumpe 1 wird das einzuführende Schlauchstück zunächst über die Fixiereinrichtung an den beiden Fixierstellen 39 und 40 auf dem Panel 23 fixiert. Das Schlauchstück zwischen den Fixiereinrichtungen 39 und 40 weist (aufgrund des Eigendralls des Schlauchstücks) dann die Form einer Schlaufe auf. Anschließend wird das Schlauchstück in die Schneckenspindel 26 eingelegt. Anschließend wird die Pumpe in Gang gesetzt, wodurch der Antriebsmotor 7 die Trägerscheibe 8 in Rotation versetzt. Gleichzeitig setzt der Spindelantrieb 28 die Schneckenspindel 26 in Drehung. Hierfür ist der Spindelantrieb 28 mit der Steuerung des Antriebsmotors 7 gekoppelt. Durch die Drehung der Schneckenspindel 26 wird der Schlauch von der Schneckenspindel 26 nach unten in Richtung der Trägerscheibe 8 geführt. Durch die Drehung der Trägerscheibe wird eine der Führungsrollen 11 auf den Schlauch zubewegt und der Schlauch greift in die Nut 34 am Außenumfang der Führungsrolle 11 ein. Durch die weitere Drehung der Trägerscheibe 8 bewegt sich die darauf angeordnete Führungsrolle 11 in Förderrichtung der Pumpe weiter und zieht dabei den Schlauch durch Kraftschluss in der Nut 34 zum einen nach unten in Richtung der Trägerscheibe 8 und drückt ihn zum anderen in radialer Richtung nach außen gegen das Gegenlager 4. Bei weiterer Drehung der Trägerscheibe 8 zieht die Führungsrolle 11 den Schlauch aufgrund der Haftreibung an der Schlauchoberfläche und des Kraftschlusses in der Nut 34 an ihrem Außenumfang weiter in die Schlauchpumpe entlang des Innenumfangs des kreissegmentförmig ausgebildeten Gegenlagers 4 ein, bis die Trägerscheibe mit der darauf angeordneten Führungsrolle 11 (nahezu) eine volle Umdrehung ausgeführt hat und der Schlauch durch die weitere Drehung der Trägerscheibe vollständig in die Schlauchpumpe eingezogen worden ist. Durch die Drehung der Trägerscheibe wird der Schlauch schließlich von der auf der Trägerscheibe 8 der Führungsrolle 11 folgenden Quetschrolle 3 schließlich gegen das Gegenlager 4 gequetscht. Auf diese Weise wird der Schlauch selbsttätig zwischen dem Außenumfang der Quetschrollen 3 und dem Gegenlager 4 eingeführt und bei weiterer Drehung der Trägerscheibe 8 zur Förderung der darin geführten Flüssigkeit gequetscht.

Ist der Schlauch vollständig in der Schlauchpumpe eingeführt, drücken die Quetschrollen 3 den Schlauch bei laufender Schlauchpumpe (also bei rotierender Trägerscheibe 8 und rotierenden Quetschrollen 3) unter Quetschung des Schlauchdurchmessers gegen die Wirkfläche 4a des Gegenlagers 4, um dadurch das in dem Schlauch geführte Medium in Förderrichtung (d.h. in Drehrichtung der Trägerscheibe 8) weiterzufördern.

Nach Beendigung des Pumpvorgangs kann die Einfädeleinrichtung bei einem erforderlichen Schlauchwechsel auch zum Ausfädeln des verbrauchten Schlauchs verwendet werden. Hierzu wird der Spindelantrieb 28 bei laufender Schlauchpumpe in umgekehrter Drehrichtung betrieben. Dadurch zieht die Schneckenspindel 26 das in die Schlauchpumpe eingelegte Schlauchstück nach oben, so dass der Eingriff des Schlauchs in der Nut 34 der Führungsrollen 11 gelöst wird. Nach einer vollen Umdrehung der Trägerscheibe ist der Schlauch vollständig aus der Schlauchpumpe heraus gezogen und kann nach Lösen der Fixierungen an den Fixierstellen 39 und 40 entnommen und durch einen neuen Schlauch ersetzt werden. Zur Einleitung der Ausfädelung eines verbrauchten Schlauchs ist eine Steuerroutine in der Steuerung des Spindelantriebs 28 vorgesehen, welche auf Knopfdruck vom Bediener ausgelöst werden kann.

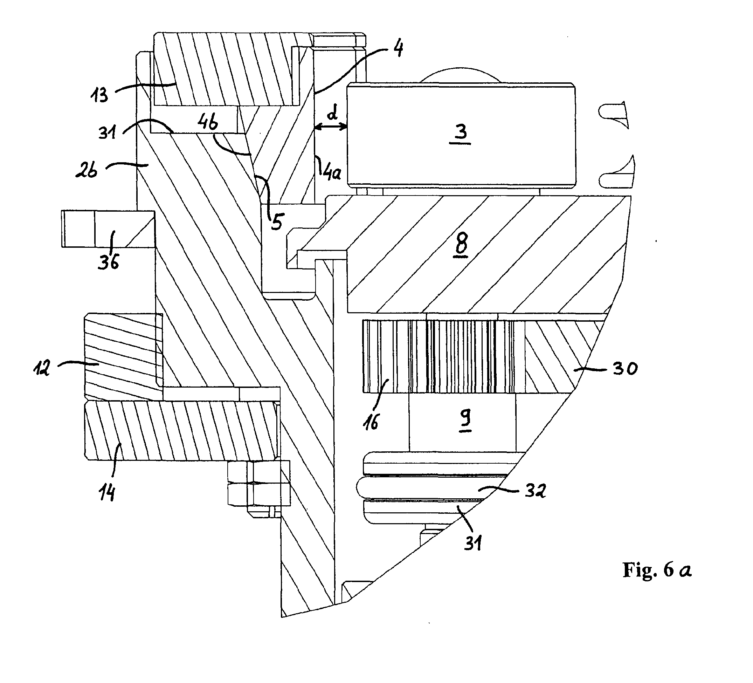

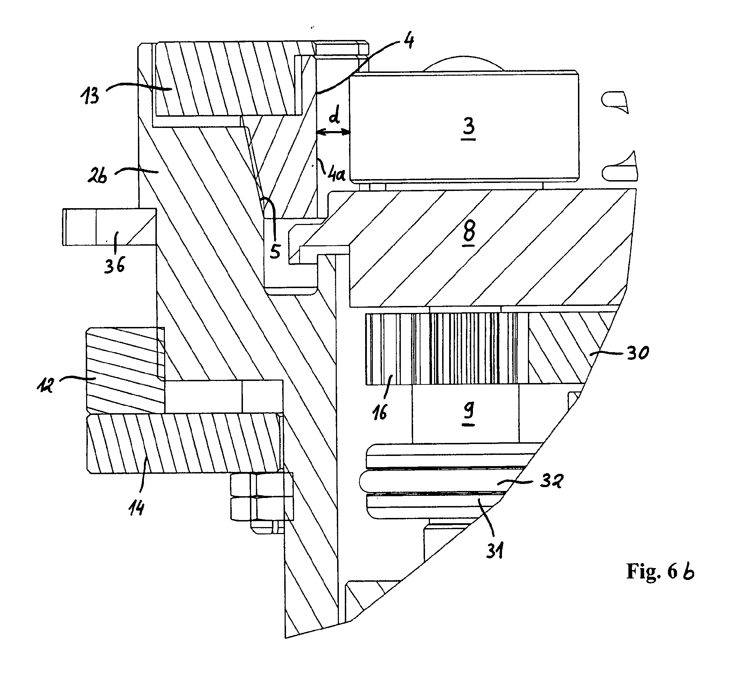

Zur optimalen Einstellung des Abstands zwischen dem Gegenlager 4 und den Quetschrollen 3 ist das Gegenlager in einem bevorzugten Ausführungsbeispiel mit seiner Wirkfläche 4a gegenüber den Quetschrollen 3 verschiebbar am Gehäuse 2 angeordnet. Hierfür ist das Gegenlager 4 mit einem Druckring 13 verbunden. Bei dem Druckring 13 handelt es sich ebenfalls um einen kreissegmentformigen Ring. Das Gegenlager 4 weist eine der Wirkfläche 4a gegenüberliegende Stellfläche 4b auf. Diese ist konisch bzw. kegelförmig ausgebildet. Die aus dem Gegenlager 4 und dem Druckring 13 bestehende Anordnung ist in der oberen Öffnung des Gehäuses 2 so angeordnet, dass sich die konische Stellfläche 4b des Gegenlagers 4 gegen eine komplementär (also ebenfalls konisch bzw. kegelförmig) geformte Stützfläche 5 am Gehäuse 2 abstützt, wobei sich die Stützfläche 5 am Gehäuse 2 nach unten (also ins Gehäuseinnere) hin konisch erweitert (Figur 6, jeweils links oben).

Auf der Außenseite des Gehäuses 2 ist ein am Gehäuse befestigter Befestigungsring 36 (welche in Figur 3 aus Gründen der Übersichtlichkeit weg gelassen worden ist) mit Befestigungsflanschen 37 zur Befestigung des Gehäuses 2 am Panel 23 des Injektionskopfs 20 vorgesehen. Auf der Außenseite des Gehäuses 2 ist weiterhin im Übergangsbereich zwischen dem unteren Gehäuseteil 2a und dem oberen Gehäuseteil 2b ein Stellring 12 angeordnet. Bei dem Stellring 12 handelt es sich um einen Kreisring, der an seiner Kreisinnenfläche über ein Innengewinde verfügt. Auf der Außenseite des Gehäuses 2 ist ein zu diesem Innengewinde komplementäres Außengewinde vorgesehen. Der Stellring ist über diese Gewindeanordnung an das Gehäuse 2 derart gekoppelt, dass durch eine Verdrehung des Stellrings 12 gegenüber dem Gehäuse 2 der Stellring in axialer Richtung kontinuierlich zwischen einer oberen Grenzstellung und einer unteren Grenzstellung bezüglich des Gehäuses 2 verschiebbar ist. Zum Verdrehen des Stellrings 12 gegenüber dem Gehäuse 2 sind am Außenumfang des Stellrings 12 mehrere Bohrungen 33 vorgesehen, in die ein Stift eingreifen kann.

An der Unterseite des Stellrings 12 liegt ein Verschiebering 14 an. Der Verschiebering 14 ist aus zwei halbkreisförmigen Ringsegmenten 14a und 14b zusammengesetzt und über mehrere Bolzen 29 mit dem Druckring 13 verbunden (Figur 3).

Über die Anordnung, bestehend aus dem Gegenlager 4, dem Druckring 13, dem Verschiebering 14 und dem Stellring 12 kann der Abstand d zwischen den Quetschrollen 3 und der Wirkfläche 4a des Gegenlagers 4 eingestellt werden.

Um den Abstand d zwischen dem Außenumfang der Quetschrollen 3 und der Wirkfläche 4a zu maximieren, wird das Gegenlager 4 in seine erste (obere) Grenzstellung gebracht (Figur 6a). Hiervon ausgehend, kann der Abstand d verkleinert werden, indem der Stellring 12 am Gehäuse 2 in Richtung seiner unteren Grenzstellung gedreht wird. Dadurch verschiebt sich der Stellring 12 von seiner oberen Grenzstellung nach unten. Dadurch wird auch der an der Unterseite des Stellrings 12 anliegende Verschiebering 14 gegenüber dem Gehäuse nach unten verschoben. Da der Verschiebering 14 über die Bolzen 29 mit dem Druckring 13 verbunden ist, verschiebt sich dadurch auch der Druckring 13 mit dem daran befestigten Gegenlager 4 nach unten. Dabei gleitet die Stellfläche 4b des Gegenlagers 4 auf der kegelförmigen Stützfläche 5 am Gehäuse 2 entlang. Bei dieser Bewegung zieht sich das kreissegmentformige Gegenlager 4 leicht zusammen und verringert ihren Durchmesser, wodurch die Wirkfläche 4a in radialer Richtung auf die Quetschrollen 3 bzw. die Führungsrollen 11 zu gedrückt wird. Durch diese Bewegung verringert sich der Abstand d zwischen der Wirkfläche 4a und dem Außenumfang der Quetschrollen 3. Ist der Stellring 12 bei weiterer Drehung in seiner unteren Grenzstellung angelangt, sitzt die Unterseite des Druckrings 13 auf einem Sockel 31 des Gehäuses 2 auf. In dieser Stellung ist der Abstand d zwischen der Wirkfläche 4a und dem Außenumfang der Quetschrollen 3 bzw. der Führungsrollen 11 in Minimalstellung (Figur 6b).

Durch diese Anordnung des Gegenlagers 4 kann das Spaltmaß (also der Abstand d) zwischen der Wirkfläche 4a und dem Außenumfang der Quetschrollen 3 auf einen für den Betrieb der Pumpe optimalen Wert eingestellt werden. Diese Einstellung erfolgt erstmalig vor der Inbetriebnahme der Schlauchpumpe. Zur Einstellung eines gewünschten Abstand d wird zweckmäßig eine Lehre verwendet, deren Dicke dem einzustellenden Spaltmaß entspricht und welche zwischen der Wirkfläche 4a und dem Außenumfang der Quetschrollen 3 eingelegt wird. Anschließend wird der Stellring 12 gegenüber dem Gehäuse verdreht, bis die Wirkfläche 4a und der Außenumfang der Quetschrollen 3 an den Außenflächen der Lehre anliegen. Das Spaltmaß kann im Bedarfsfall bei einer Wartung der Schlauchpumpe nachgestellt werden.

Die Erfindung ist nicht auf das beschriebene Ausführungsbeispiel beschränkt. So kann die Erfindung nicht nur in Radial-Schlauchpumpen sondern bspw. auch in Schlauchpumpen mit einem linearen Wirksystem, wie z.B. in sog. linearen Peristaltikpumpen bzw. Wanderwellenpumpen, verwendet werden. Der Einsatz der erfindungsgemäßen Schlauchpumpe ist ferner nicht auf Injektionsvorrichtungen beschränkt, sondern erstreckt sich auch auf andere Pumpeinrichtungen, wie z.B. Infusionspumpen.

## Patentansprüche

1. Schlauchpumpe (1) zur Förderung eines in einem Schlauch geführten Mediums, mit einem Gehäuse (2) und mehreren Quetschelementen (3), welche den Schlauch unter Quetschung des Schlauchs gegen die Wirkfläche (4a) eines Gegenlagers (4) drücken und dadurch das Medium in dem Schlauch in Förderrichtung weiter fördern, wobei das Gegenlager (4) über eine konische oder kegelförmige Stellfläche (4b) verfügt, welche sich gegen eine komplementär geformte Stützfläche (5) am Gehäuse (2) abstützt und der Abstand (d) zwischen den Quetschelementen (3) und der Wirkfläche (4a) des Gegenlagers (4) durch Verschieben des Gegenlagers (4) gegenüber dem Gehäuse (2) entlang der Stützfläche (5) einstellbar ist, **dadurch gekennzeichnet, dass** das Gegenlager (4) als Ringsegment ausgebildet ist.

2. Schlauchpumpe nach Anspruch 1, **dadurch gekennzeichnet, dass** die Quetschelemente durch Quetschrollen (3) gebildet sind, welche über ein Getriebe (6) von einem Antriebsmotor (7) in Rotation versetzt werden.

3. Schlauchpumpe nach Anspruch 2, **dadurch gekennzeichnet, dass** die Quetschrollen (3) auf einer Trägerscheibe (8) gelagert sind, wobei die Achse (9) jeder Quetschrolle (3) parallel zur Antriebswelle (10) des Antriebsmotor (7) verläuft.

4. Schlauchpumpe nach Anspruch 3, **dadurch gekennzeichnet, dass** auf der Trägerscheibe (8) zwischen zwei benachbarten Quetschrollen (3) jeweils eine Führungsrolle (11) angeordnet ist.

5. Schlauchpumpe nach einem der voranstehenden Ansprüche, **dadurch gekennzeichnet, dass** das Gegenlager (4) an einen Stellring (12) gekoppelt ist, welcher am Gehäuse (2) angeordnet und in axialer Richtung relativ zum Gehäuse (2) zwischen einer oberen Grenzstellung und einer unteren Grenzstellung verschiebbar ist.

6. Schlauchpumpe nach Anspruch 5, **dadurch gekennzeichnet, dass** der Stellring (12) über ein Gewinde an das Gehäuse (2) gekoppelt ist und durch Verdrehen des Stellrings (12) gegenüber dem Gehäuse (2) dieser in axialer Richtung kontinuierlich zwischen der oberen Grenzstellung und der unteren Grenzstellung verschiebbar ist.

7. Schlauchpumpe nach einem der voranstehenden Ansprüche, **dadurch gekennzeichnet, dass** das Gegenlager (4) mit einem Druckring (13) verbunden ist, welcher an einem an dem Stellring (12) anliegenden Verschiebering (14) befestigt ist.

8. Schlauchpumpe nach Anspruch 7, **dadurch gekennzeichnet, dass** sich der Verschiebering (14) bei Verschieben bzw. Verdrehen des Stellrings (12) gegenüber dem Gehäuse (2) in axialer Richtung gegenüber dem Gehäuse bewegt und dabei die konische Stellfläche (4b) des Gegenlagers (4) auf der Stützfläche (5) des Gehäuses verschiebt.

9. Schlauchpumpe nach Anspruch 8, **dadurch gekennzeichnet, dass** die konische Stellfläche (4b) des Gegenlagers (4) auf der Stützfläche (5) des Gehäuses zwischen einer ersten Grenzstellung und einer zweiten Grenzstellung verschiebbar ist, wobei die erste Grenzstellung zu der der oberen Grenzstellung des Stellrings (12) und die zweite Grenzstellung zu der unteren Grenzstellung des Stellrings (12) korrespondiert.

10. Schlauchpumpe nach Anspruch 9, **dadurch gekennzeichnet, dass** der Abstand (d) zwischen dem Außenumfang der Quetschrollen (3) und der Wirkfläche (4a) des Gegenlagers (4) in der ersten Grenzstellung der Stellfläche (4a) einen Maximalwert und in der zweiten Grenzstellung der Stellfläche (4a) einen Minimalwert einnimmt.

11. Schlauchpumpe nach einem der Ansprüche 7 bis 10, **dadurch gekennzeichnet, dass** der Verschiebering (14) von zwei oder mehr Ringsegmenten (14a, 14b) gebildet ist.

12. Schlauchpumpe nach Anspruch 1, **dadurch gekennzeichnet, dass** sich das Ringsegment des Gegenlagers (4) über einen Winkelbereich von 200° bis 300° erstreckt.

13. Schlauchpumpe nach Anspruch 1, **dadurch gekennzeichnet, dass** sich der Durchmesser des Ringsegments des Gegenlagers (4) verringert, wenn das Gegenlager (4) von seiner ersten Grenzstellung in seine zweite Grenzstellung verschoben wird.

## Claims

1. Tube pump (1) for conveying a medium carried in a tube, with a housing (2) and a plurality of squeezing elements (3) which press the tube against the working surface (4a) of an abutment (4) and in so doing squeeze the tube and convey the medium in the tube further in the conveying direction, wherein the abutment (4) has a conical or tapering adjustment surface (4b) which is supported on the supporting surface (5) of complementary shape on the housing (2) and the distance (d) between the squeezing elements (3) and the working surface (4a) of the abutment (4) is adjustable by displacing the abutment (4) in relation to the housing (2) along the supporting surface (5), **characterised in that** the abutment (4) is embodied as a ring segment.

2. Tube pump according to claim 1, **characterised in that** the squeezing elements are formed by squeezing rollers (3) which are set in rotation by a driving motor (7) through a gear unit (6).

3. Tube pump according to claim 2, **characterised in that** the squeezing rollers (3) are mounted on a carrier disk (8), the shaft (9) of each squeezing roller (3) running parallel to the driving shaft (10) of the driving motor (7).

4. Tube pump according to claim 3, **characterised in that** in each case a guiding roller (11) is arranged on the carrier disk (8) between two neighbouring squeezing rollers (3).

5. Tube pump according to one of the preceding claims, **characterised in that** the abutment (4) is coupled to an adjusting ring (12) which is arranged on the housing (2) and displaceable in the axial direction relative to the housing (2) between an upper limit position and a lower limit position.

6. Tube pump according to claim 5, **characterised in that** the adjusting ring (12) is coupled to the housing (2) by means of a thread and the adjusting ring is continuously displaceable between the upper limit position and the lower limit position by turning the adjusting ring (12) in relation to the housing (2).

7. Tube pump according to one of the preceding claims, **characterised in that** the abutment (4) is connected to a thrust ring (13) which is fastened to a displacement ring (14) which bears on the adjusting ring (12).

8. Tube pump according to claim 7, **characterised in that** when the adjusting ring (12) is displaced or turned in relation to the housing (2), the displacement ring (14) moves in an axial direction relative to the housing and in the process displaces the conical adjustment surface (4b) of the abutment (4) on the supporting surface (5) of the housing.

9. Tube pump according to claim 8, **characterised in that** the conical adjustment surface (4b) of the abutment (4) is displaceable on the supporting surface (5) of the housing between a first limit position and a second limit position, the first limit position corresponding to the upper limit position of the adjusting ring (12) and the second limit position to the lower limit position of the adjusting ring (12).

10. Tube pump according to claim 9, **characterised in that** the distance (d) between the external circumference of the squeezing rollers (3) and the working surface (4a) of the abutment (4) assumes a maximum value in the first limit position of the adjustment surface (4b) and a minimum value in the second limit position of the adjustment surface (4b).

11. Tube pump according to one of claims 7 to 10, **characterised in that** the displacement ring (14) is formed by two or more ring segments (14a, 14b).

12. Tube pump according to claim 1, **characterised in that** the ring segment of the abutment (4) extends over an angular range of 200° to 300°.

13. Tube pump according to claim 1, **characterised in that** the diameter of the ring segment of the abutment (4) reduces when the abutment (4) is displaced from its first limit position to its second limit position.

## Revendications

1. Pompe péristaltique (1) servant à acheminer un milieu guidé dans un tuyau flexible, comprenant un boîtier (2) et plusieurs éléments de compression (3), lesquels appuient sur le tuyau flexible en compressant ce dernier contre la surface active (4a) d'une butée (4) et poursuivent ce faisant l'acheminement du milieu dans le tuyau flexible dans la direction d'acheminement, sachant que la butée (4) dispose d'une surface de réglage (4b) conique ou effilée, laquelle s'appuie au niveau du carter (2) contre une surface d'appui (5) formée de manière complémentaire et sachant que la distance (d) entre les éléments de compression (3) et la surface active (4a) de la butée (4) peut être réglée en déplaçant par coulissement la butée (4) par rapport au carter (2) le long de la surface d'appui (5), **caractérisée en ce que** la butée (4) est réalisée sous la forme d'un segment annulaire.

2. Pompe péristaltique selon la revendication 1, **caractérisée en ce que** les éléments de compression sont formés par des galets de compression (3), lesquels sont amenés en rotation par un moteur d'entraînement (7) par l'intermédiaire d'une transmission (6).

3. Pompe péristaltique selon la revendication 2, **caractérisée en ce que** les galets de compression (3) sont logés sur un disque de support (8), sachant que l'axe (9) de chaque galet de compression (3) s'étend de manière parallèle par rapport à l'arbre d'entraînement (10) du moteur d'entraînement (7).

4. Pompe péristaltique selon la revendication 3, **caractérisée en ce que** respectivement un galet de guidage (11) est disposé sur le disque de support (8) entre deux galets de compression (3) adjacents.

5. Pompe péristaltique selon l'une quelconque des revendications précédentes, **caractérisée en ce que** la butée (4) est couplée à une bague de réglage (12), laquelle est disposée au niveau du carter (2) et peut être déplacée par coulissement dans la direction axiale, par rapport au carter (2), entre une position limite supérieure et une position limite inférieure.

6. Pompe péristaltique selon la revendication 5, **caractérisée en ce que** la bague de réglage (12) est couplée au carter (2) par l'intermédiaire d'un filetage et peut être déplacée par coulissement par rotation de la bague de réglage (12) par rapport au carter (2) dans la direction axiale en continu entre la position limite supérieure et la position limite inférieure.

7. Pompe péristaltique selon l'une quelconque des revendications précédentes, **caractérisée en ce que** la butée (4) est reliée à une bague de pression (13), laquelle est fixée au niveau d'une bague de déplacement par coulissement (14) reposant au niveau de la bague de réglage (12).

8. Pompe péristaltique selon la revendication 7, **caractérisée en ce que** la bague de déplacement par coulissement (14) se déplace par rapport au carter dans la direction axiale lors du déplacement par coulissement ou lors de la rotation de la bague de réglage (12) par rapport au carter (2) et déplace par coulissement dans ce cadre la surface de réglage (4b) conique de la butée (4) sur la surface d'appui (5) du carter.

9. Pompe péristaltique selon la revendication 8, **caractérisée en ce que** la surface de réglage (4b) conique de la butée (4) peut être déplacée par coulissement sur la surface d'appui (5) du carter entre une première position limite et une deuxième position limite, sachant que la première position limite correspond à la position limite supérieure de la bague de réglage (12) et que la deuxième position limite correspond à la position limite inférieure de la bague de réglage (12).

10. Pompe péristaltique selon la revendication 9, **caractérisée en ce que** la distance (d) entre la périphérie extérieure des galets de compression (3) et la surface active (4a) de la butée (4) prend une valeur maximale dans la première position limite de la surface de réglage (4a) et prend une valeur minimale dans la deuxième position limite de la surface de réglage (4a).

11. Pompe péristaltique selon l'une quelconque des revendications 7 à 10, **caractérisée en ce que** la bague de déplacement par coulissement (14) est formée par deux segments annulaires ou plus (14a, 14b).

12. Pompe péristaltique selon la revendication 1, **caractérisée en ce que** le segment annulaire de la butée (4) s'étend sur une plage angulaire allant de 200° à 300°.

13. Pompe péristaltique selon la revendication 1, **caractérisée en ce que** le diamètre du segment annulaire de la butée (4) diminue, lorsque la butée (4) est déplacée par coulissement depuis sa première position limite dans sa deuxième position limite.
